# FASCICULE DE BREVET EUROPEEN

(11) **EP 1 781 115 B1**
(45) Date de publication et mention de la délivrance du brevet: **12.11.2008**
(21) Numéro de dépôt: 05793683.3
(22) Date de dépôt: 26.07.2005
(51) Int. Cl.: A23K 1/16, A23K 1/00, A61K 31/045, A61K 31/047, A61K 31/19

(54) **COMPLEMENT ALIMENTAIRE POUR ANIMAUX D'ELEVAGE**
FUTTERMITTELERGÄNZUNG
FOOD SUPPLEMENT FOR LIVESTOCK

(30) Priorité: 13.08.2004 FR 0408893
(43) Date de publication de la demande: 09.05.2007
(73) Titulaire: Universite de Rennes 1, 35065 Rennes Cedex 7 (FR)
(72) Inventeur: LEGRAND, Alain, F-22270 Jugon-les-Lacs (FR); MITRE, Romain, F-32600 L'Isle Jourdan (FR)
(74) Mandataire: Larcher, Dominique
(86) Numéro de dépôt international: PCT/FR2005/001945
(87) Numéro de publication internationale: WO 2006/024742

(56) Documents cités:
- WO-A-97/44352
- US-A- 4 423 072
- US-A- 5 660 852

## Description

L'invention concerne le domaine de l'élevage agricole.

Plus précisément, l'invention concerne le domaine de la conception et de la fabrication des compléments alimentaires destinées aux mammifères d'élevage. L'invention trouve ainsi tout particulièrement, mais non exclusivement, son application dans le cadre de l'élevage porcin.

L'état sanitaire des élevages est l'une des préoccupations majeures des éleveurs et il est important de mettre en oeuvre des moyens permettant d'assurer un excellent état sanitaire des animaux.

Parallèlement, la législation européenne va interdire dès 2012 les traitements préventifs des maladies infectieuses par antibiothérapie dans les élevages.

Un objectif de la présente invention est de proposer un complément alimentaire et un procédé non thérapeutique de mise en oeuvre de celui-ci permettant de concourir de façon préventive à l'obtention dans les élevages d'un état sanitaire satisfaisant des animaux, tout en s'affranchissant de l'utilisation à cette fin de molécules antibiotiques.

On sait par ailleurs que chez les mammifères, le colostrum (secrétions mammaires du jour de la mise bas et des quelques jours suivants) joue un rôle fondamental dans la survie des petits et que le lait joue également un rôle capital dans le développement de ceux-ci.

Par exemple, les porcelets naissent sans défenses immunitaires et le rôle du colostrum, très riche en anticorps, est de leur apporter des défenses immunitaires dites « passives » transmises par leur mère. La transmission d'anticorps aux porcelets se poursuit ensuite par le lait.

Un autre objectif de la présente invention est donc de proposer un complément alimentaire permettant d'enrichir la composition du colostrum puis du lait des mammifères auxquels il est administré afin d'augmenter et/ou stimuler les défenses immunitaires de leurs petits.

Un autre objectif de la présente invention est de décrire un tel complément alimentaire qui puisse être obtenu facilement à partir d'un produit naturel.

Ces différents objectifs sont atteints grâce à l'invention qui concerne un complément alimentaire pour mammifères d'élevage en période de gestation puis, le cas échéant en période de lactation destiné à modifier la composition lipidique et immunologique de leur colostrum puis le cas échéant de leur lait caractérisé en ce qu'il comprend un extrait assimilable d'huile de foie de poisson(s) contenant au moins un composé *sn*-1-O-alkylglycérol.

Les composés *sn*-1-O-alkyglycérols, ci-après parfois désignés par l'abréviation 1-O-AKG, sont des éther-lipides, plus précisément des éthers de glycérol de formule générale (I) : 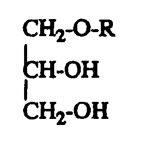 dans laquelle R représente un radical « alkyl » constitué d'une chaîne hydrocarbonée comprenant de 14 à 18 atomes de carbone et pouvant présenter une (ou plusieurs) insaturations.

On notera que l'on connaissait dans l'état de la technique, par WO-A-97/44352, un complément alimentaire pour animaux comprenant de l'huile de requin en tant que véhicule pour une composition contenant des séquences d'amino-acides non naturels similaires à ceux d'une hormone animale naturelle.

On connaissait également, par US-A-5,660,852 un complément alimentaire pour ruminant destiné à traiter un déséquilibre nutritionnel et provoquer une mobilisation des graisses.

L'activité immunostimulante de ces composés chez les rongeurs à été démontrée *in vitro* (Yamamoto, N. & Ngwenya, B. Z. (1987) Activation of mouse peritoneal macrophages by lysophospholipids and ether derivatives of neutral lipids and phospholipids. Cancer Res 47: 2008-2013 ; Lininan, J. W., Long, M. J., Korst, D. R & Bethell, F. H. (1959) Studies on the stimulation of hemopoiesis by batyl alcohol. J Lab Clin Med 54: 335-343 ; Homma, S. & Yamamoto, N. (1990) Activation process of macrophages after in vitro treatment of mouse lymphocytes with dodecylglycerol. Clin Exp Immunol 79: 307-313) et *in vivo* (Oh, S. Y. & Jadhav, L. S. (1994) Effects of dietary alkylglycerols in lactating rats on immune responses in pups. Pediatr Res 36: 300-305 ; Chorostowska-Wynimko, J., Krotkiewski, M., Radomska-Lesniewska, D., Sokolnicka, I. & Skopinska-Rozewska, E. (2001) The synergistic effect of lactic acid bacteria and alkylglycerols on humoral immunity in mice. Int J Tissue React 23: 81-87).

Ces études menées chez les rongeurs portent toujours sur des molécules de synthèse administrées, au mieux au début de la lactation qui n'influencent donc pas l'immunité passive apportée par le colostrum.

On notera par ailleurs qu'il a déjà été proposé dans l'état de la technique des compléments alimentaires pour truies incluant des huiles de foie de poisson. (Rooke, J. A., Sinclair, A. G. & Ewen, M. (2001) Changes in piglet tissue composition at birth in response to increasing maternal intake of long-chain n-3 polyunsaturated fatty acids are non-linear. Br J Nutr 86: 461-470 ; Rooke, J. A., Sinclair, A. G. & Edwards, S. A. (2001) Feeding tuna oil to the sow at different times during pregnancy has different effects on piglet long-chain polyunsaturated fatty acid composition at birth and subsequent growth. Br J Nutr 86: 21-30).

Toutefois, ces études mettent en oeuvre des huiles de foie de poisson ne contenant pas de *sn*-1-O-alkylglycérols. Or, de telles huiles sont connues pour leurs effets inhibiteurs de l'immunité (Calder, P. C., Yaqoob, P., Thies, F., Wallace, F. A. & Miles, E. A. (2002) Fatty acids and lymphocyte functions. Br J Nutr 87 Suppl 1: S31-48 ; Calder, P. C. (1998) Dietary fatty acids and the immune system. Nutr Rev 56: S70-83 ; Fritsche, K. L., Huang, S. C. & Misfeldt, M. (1993) Fish oil and immune function. Nutr Rev 51: 24 ; Yaqoob, P. (1998) Lipids and the immune response. Curr Opin Clin Nutr Metab Care 1: 153-161).

Dans le cadre de la présente invention, l'huile de foie de poisson utilisée comprend en tant que composé(s) *sn*-1-O-alkylglycérol du 16:1n-7 *sn-*1-O-alkylglycérol de formule (II) :

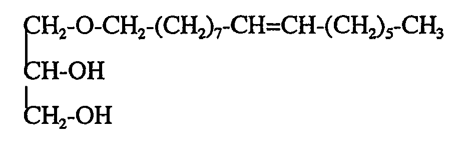

Avantageusement, cette huile contient un mélange de 16:1n-7 *sn-*1-O-alkylglycérol (II) 16:0 *sn*-1-O-alkylglycérol (de formule III), 18:1n-9 *sn*-1-O-alkylglycérol (de formule IV) et 18: 1n-7 *sn*-1-O-alkylglycérol (de formule V).

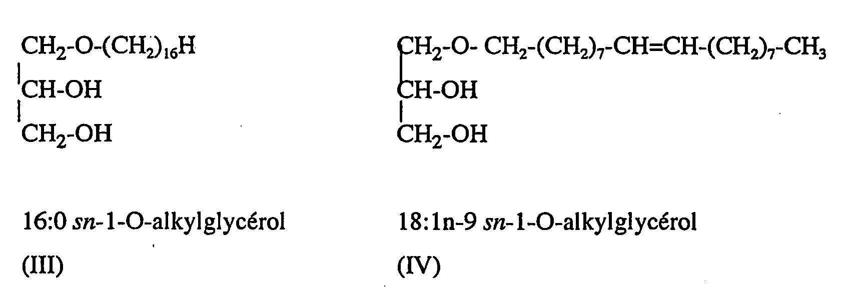

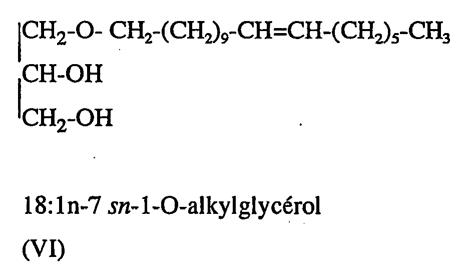

Différents poissons pourront être utilisés pour fournir l'huile de foie de poisson(s) nécessaire à la mise en oeuvre de la présente invention dès lors que leur foie titrera en composés *sn*-1-O-alkylglycérol(s). Préférentiellement, on utilisera une huile de foie de requin(s) tel que *Squalus Acanthias, Somniosus microcephalus*, ou encore *Centrophorus squamosus*. D'une façon préférée entre toutes, on utilisera comme source le requin Siki (*Centrophorus squamosus*).

Egalement préférentiellement, l'huile de foie de poisson(s) utilisée dans le cadre de la présente invention titre à au moins 20 % en poids en composé(s) *sn-*1-O-alkylglycérol(s).

Comme indiqué ci-dessus, l'invention pourra être mise en oeuvre pour l'élevage des mammifères de rapport. Préférentiellement, le complément alimentaire selon l'invention sera toutefois destiné aux truies.

L'invention, ainsi que les différents avantages qu'elle présente, seront plus facilement compris grâce à la description qui va suivre d'un mode de réalisation de celle-ci.

Dans le cadre du présent mode de réalisation, on a utilisé un extrait d'huile de foie de requin Siki (*Centrophorus Squamosus*) contenant 25 % en poids de composés *sn*-1-O-alkylglycérols. Le squalène contenu dans l'huile brute a été préalablement extrait de celle-ci de façon à obtenir une huile désqualènisée constituant un complément alimentaire selon la présente invention.

Cet extrait d'huile de foie de requin Siki présentait la composition pondérale en *sn*-1-O-alkylglycérols figurant dans le tableau I ci-après, et la composition pondérale en acides gras figurant dans la deuxième colonne du tableau II ci-après :

**Tableau I**

| Composé 1-O-AKG | Pourcentage pondéral du complément |
|---|---|
| C14:0 | 3% |
| C16:0 | 7,2% |
| C16:1 n-7 | 14,9% |
| C18:0 | 1,2% |
| C18:1 n-7 | 8,3% |
| C18:1n-9 | 58,8% |
| Autres espèces | Moins de 1% par composé |

Ce complément alimentaire a été administré à un lot de 12 truies de la lignée génétique Large White X Landrace (« Lot C »), en complément à des aliments classique de gestation puis de lactation, à raison d'une dose journalière de 32 g par jour et par truie.

Ce même aliment classique a par ailleurs été distribué dans les mêmes quantités et en l'absence du complément alimentaire selon la présente invention à un lot témoin (« Lot T ») de 12 autres truies de la même lignée.

Le complément alimentaire selon l'invention a été distribué aux truies du Lot C en le répartissant grâce à une seringue sur l'aliment.

Ce complément alimentaire a été distribué aux truies du lot C pendant 5 semaines avant la mise bas et jusqu'au sevrage soit 28 jours après la mise bas.

Durant la période de gestation, l'aliment de gestation a été distribué à raison de 2,7 kilogrammes par jour en un seul repas.

Durant la période de lactation, l'aliment de lactation a été distribué, le jour J de la mise bas à raison de 2,7 kilogramme par jour en un seul repas.

Le premier jour suivant la mise bas (J1), l'aliment de lactation a été distribué à raison de 3,5 kilogrammes en 2 repas.

Du deuxième au sixième jour (J2 à J6), suivant la mise bas l'aliment de lactation a été distribué à raison de 4,5 kilogrammes par jour en deux repas.

Du septième jour et jusqu'au sevrage (J7 à J28), l'aliment de lactation a été distribué à raison de 5,5 kilogrammes par jour en deux repas.

Les compositions en acides gras de l'aliment classique de gestation et de l'aliment classique de lactation sont données dans la troisième et la quatrième colonnes du tableau II ci-après.

**Tableau II**

| Acides gras (%) | Huile de foie de requin | Aliment distribué pendant la gestation | Aliment distribué pendant la gestation |
|---|---|---|---|
| 14:0 | 1.5 | - | - |
| 16:0 | 16.5 | 14.5 | 14.8 |
| 16:1 n-7 | 4.1 | - | - |
| 18:0 | 2.2 | 2.5 | 2.5 |
| 18:1 n-9 | 34.2 | 22.8 | 23.9 |
| 18:1 n-7 | 5.6 | 1.4 | 1.4 |
| 18:2 n-6 | - | 51.5 | 50.5 |
| 20:0 | 0.3 | - | - |
| 18:3 n-3 | - | 4.8 | 4.7 |
| 18:4 n-3 | 11.4 | - | - |
| 20:4 n-3 | 10.4 | - | - |
| 20:5 n-3 | 4.9 | - | - |
| 22:5 n-3 | 4.4 | - | - |
| Autres | 4.5 | 2.5 | 2.2 |
| % de l'aliment (en poids sec) | - | 3.9 | 3.9 |

Les prélèvements suivants ont été effectuées chez les truies et les porcelets :
- prises de sang sur les truies avant la mise en lots, la veille de la mise bas et 14 jours après la mise bas ;
- prises de sang sur les porcelets, à 2 jours d'âge (J2), à 21 jours (J21) et à 36 jours d'âge, c'est-à-dire après le sevrage (J36).
- prélèvements de colostrum chez les truies 12 h après la mise bas ;
- prélèvement de lait chez les truies 14 jours (J14) et 28 jours (J28) après la mise bas, les échantillons de lait étant obtenus par traite de toutes les tétines de chaque truie, les porcelets ayant été séparés de leur mère pendant l'heure précédente.

Chaque échantillon de colostrum et de lait a été séparé en deux parties.

L'une a été congelée en vue d'une analyse de la teneur en lipides. Le lactosérum de l'autre a été extrait de façon classique puis congelé.

3 ml dé chaque échantillon de colostrum et de lait ont été centrifugés (2000 g à 4 °C pendant 20 min) afin de séparer la crème de la fraction liquide. Les lipides de la crème ont été extraits de façon classique et la fraction de lipides totaux a été pesée et dissoute dans du chloroforme.

70 mg de l'extrait de lipides totaux de chaque échantillon ont été séchés et 30 µg de 17:0 *sn*-1-O-alkylglycérol ont été ajouté à titre de standard interne. Ce mélange a été dissous dans 10 mL de KOH méthanolique.

La fraction insaponifiable de ce mélange a été extraite de façon classique. Les composés *sn*-1-O-alkylglycérols ont ensuite été séparés de cette fraction insaponifiable par chromatographie sur gel de silice.

La fraction des lipides totaux des échantillons de lait et de colostrum a fait l'objet pour chaque échantillon d'un dosage des acides gras.

Les immuno-globulines G, A et M (IGG, IGA et IGM) ont quant à eux été dosés par méthode ELISA.

Enfin, on notera que les anticorps anti-Aujeszky ont quant à eux été dosés en utilisant des kits obtenus de la société IDEXX Sarl (Cergy-Pontoise, France).

Les résultats obtenus sont donnés ci-après.

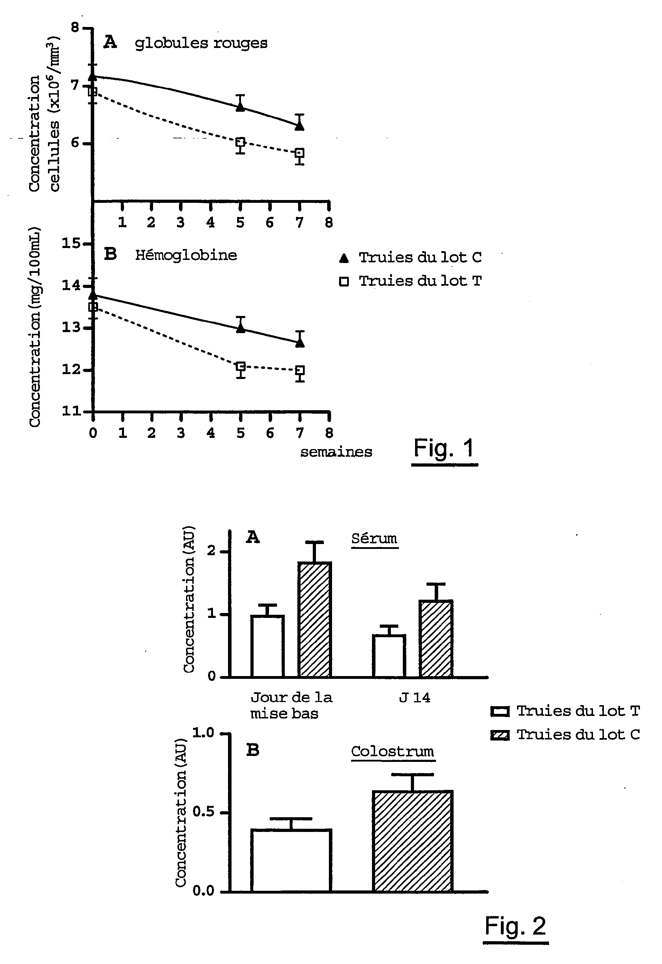

Les figures 1A et 1B représentent les effets de la mise en oeuvre du procédé susmentionné sur la concentration moyenne en globules rouges (figure 1A) et sur la concentration moyenne en hémoglobine (figure 1B) dans les échantillons de sang des 12 truies du Lot C traitées selon ledit procédé et dans les échantillons de sang des 12 truies du Lot témoin.

Ces figures 1A et 1B indiquent clairement une augmentation à la fois du nombre de globules rouges et du taux d'hémoglobine chez les truies ayant reçu le complément alimentaire selon la présente invention.

Les effets du procédé susmentionné sur la concentration en anticorps spécifiques de la maladie d'Aujeszky sont indiqués sur les Figures 2A et 2B.

La figure 2A indique le taux de ces anticorps dans le sérum sanguin des truies du Lot C et du Lot T (témoin) lors de la mise bas et 14 jours après celle-ci.

La figure 2B indique le taux de ces anticorps dans le colostrum des truies du Lot C et du Lot T (témoin).

Ces figures 2A et 2B démontrent un effet bénéfique du complément alimentaire selon l'invention sur la vaccination des truies contre la maladie d'Aujeszky puisqu'on observe une augmentation nette des anticorps destiné à lutter contre cette maladie lorsque le complément alimentaire selon l'invention est administrée aux truies.

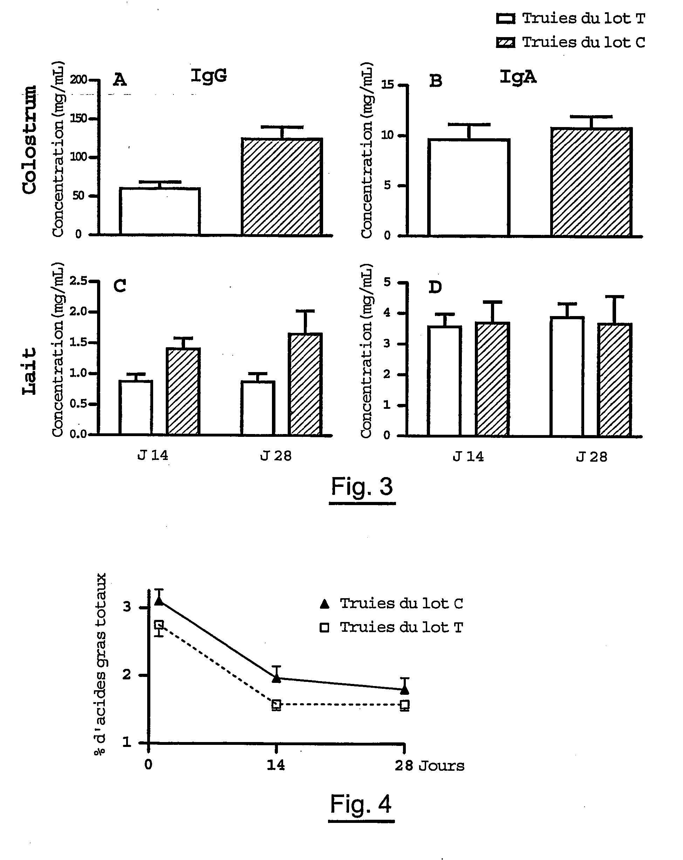

Les effets du complément alimentaire selon l'invention sur la teneur du colostrum et du lait maternel en anticorps totaux (IgG et IgA) sont indiqués à la Figure 3. Ces anticorps, avec les anti-corps de la maladie d'Aujeszky sont les vecteurs de l'immunité passive transmise par la mère à ses petits.

Cette figure 3 indique que les IgG ont été influencées positivement par le complément alimentaire selon la présente invention (les IgA ne semblent pas avoir été modifiées).

D'autre part, l'influence du complément alimentaire sur la composition du colostrum et du lait en *sn*-1-O-alkylglycérols (1-O-AKG) et en acides gras est synthétisé dans le Tableau III suivant :

**Tableau III**

| | Colostrum | | Lait 14 jours après la mise bas | | Lait 28 jours après la mise bas | |
|---|---|---|---|---|---|---|
| | Lot T | Lot C | Lot T | Lot C | Lot T | Lot C |
| 1-O-AKG en µg/ml | | | | | | |
| 14:0 | 19,17 ± 3,20 | 12,16 ± 1,78 | 22,63 ± 2,22 | 25,60 ± 2,26 | 16,18±1,60 | 22,80 ± 3.77 |
| 16:0 | 53,72 ± 6,56 | 31,58 ± 5,49* | 103,99 ± 8,79 | 109,45 ± 9,94 | 62,33 ± 3,55 | 73,82 ± 7.91 |
| 16:1 | ND | 5,06 ± 0,81*** | ND | 4,13 ± 0,30*** | ND | 3,96 ± 0.21*** |
| 18:0 | 30,00 ± 2,73 | 17,88 ± 3,63* | 32,30 ± 3,39 | 29,60 ± 4,39 | 16,34 ± 0,49 | 19,27 ± 1.77 |
| | | | | | | |
| 18:1 | 84,45 ± 7,41 | 68,40 ± 12,30 | 97,80 ± 8,45 | 106,90 ± 13,76 | 50,37 ± 2,21 | 65,36 ± 5.07 * |
| total | 187.33 ± 17.94 | 135.09 ± 23.8 | 256.71 ± 20.16 | 275.69 ± 27.87 | 145.22 ± 5.75 | 185.20 ± 17.22 * |

| Acide Gras en mg /mL | | | | | | |
|---|---|---|---|---|---|---|
| 14:0 | 0.67 ± 0.10 | 0.52 ± 0.09 | 1.56 ± 0.10 | 1.91 ± 0.11* | 1.25 ± 0.08 | 1.50 ± 0.13 |
| 16:0 | 10.72 ± 1.21 | 7.48 ± 1.44 | 17.60 ± 1.04 | 19.54 ± 1.75 | 12.82 ± 0.71 | 14.21 ± 0.87 |
| 16:1 | 0.53 ± 0.05 | 0.42 ± 0.08 | 0.38 ± 0.06 | 0.46 ± 0.08 | 0.18 ± 0.02 | 0.20 ± 0.03 |
| n-9 | | | | | | |
| 16:1 | 1.21 ± 0.18 | 1.04 ± 0.27 | 4.24 ± 0.33 | 4.90 ± 0.25 | 3.14 ± 0.24 | 3.51 ± 0.22 |
| n-7 | | | | | | |
| 18:0 | 3.04 ± 0.37 | 2.01 ± 0.44 | 4.19 ± 0.68 | 4.38 ± 0.82 | 2.15 ± 0.14 | 2.30 ± 0.18 |
| 18:1 | 15.22 ± 1.66 | 11.40 ± 2.77 | 25.69 ± 3.35 | 30.14 ± 5.04 | 14.24 ± 1.08 | 16.52 ± 1.24 |
| n-9 | | | | | | |
| 18:1 | 1.27 ± 0.14 | 0.96 ± 0.19 | 1.79 ± 0.22 | 2.14 ± 0.35 | 0.99 ± 0.07 | 1.16 ± 0.06 |
| n-7 | | | | | | |
| 18:2 | 12.89 ± 1.36 | 9.04 ± 1.37 | 11.99 ± 1.32 | 13.75 ± 2.28 | 7.54 ± 0.48 | 7.98 ± 0.48 |
| n-6 | | | | | | |
| 18:3 | 1.18 ± 0.14 | 0.86 ± 0.13 | 1.06 ± 0.11 | 1.26 ± 0.21 | 0.67 ± 0.05 | 0.73 ± 0.05 |
| n-3 | | | | | | |
| 20:1 | 0.15 ± 0.02 | 0.17 ± 0.04 | 0.30 ± 0.05 | 0.40 ± 0.07 | 0.17 ± 0.01 | 0.25 ± 0.01*** |
| n-9 | | | | | | |
| 20:2 | 0.27 ± 0.03 | 0.17 ± 0.03* | 0.37 ± 0.06 | 0.42 ± 0.09 | 0.20 ± 0.02 | 0.21 ± 0.02 |
| n-6 | | | | | | |
| 20:4 | 0.46 ± 0.05 | 0.32 ± 0.05* | 0.36 ± 0.04 | 0.43 ± 0.06 | 0.20 ± 0.02 | 0.21 ± 0.01 |
| n-6 | | | | | | |
| 22:5 | 0.21 ± 0.03 | 0.15 ± 0.02 | 0.17 ± 0.02 | 0.20 ± 0.04 | 0.08 ± 0.01 | 0.09 ± 0.01 |
| n-3 | | | | | | |

Selon les résultats figurant dans ce tableau, on constate un passage des *sn*-1-O-alkylglycérols alimentaires dans le colostrum et le lait : ce passage est illustré par la présence de l'espèce C16:1, uniquement présente dans l'huile de foie de requin, et qui joue ici le rôle de traceur.

L'étude de la répartition des acides gras au sein des différentes catégories révèle un accroissement global du pourcentage d'acides gras n-3 (ou ω3) dans le colostrum et le lait maternel comme on peut le voir sur la figure 4 qui représente l'évolution dans le temps du taux pondéral d'acides gras n-3 par rapport au total d'acide gras dans les sécrétions mammaires des truies du lot T et du lot C.

Les prises de sang réalisées sur les porcelets (5 représentatifs de chaque portée) à 2 jours (J2), 21 jours (J21) et 36 jours (J36) après la mise bas ont permis de mesurer la concentration en globules blancs totaux et en anticorps dans le sang de ces porcelet

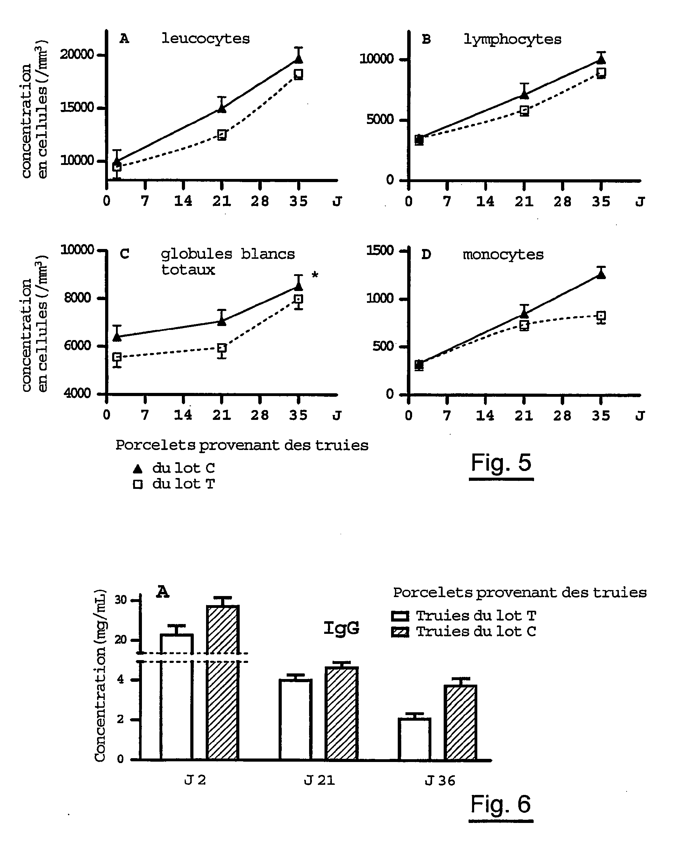

Les effets de la complémentation alimentaire des truies sur les globules blancs totaux des porcelets ainsi que sur leur répartition dans les sous-classes principales sont représentés sur la Figure 5 (panneau A : globules blancs totaux (leucocytes), panneau B : lymphocytes ; panneau C : neutrophiles ; panneau D : monocytes).

Selon cette figure 5, on peut constater une augmentation du nombre de globules blancs totaux, des neutrophiles, des lymphocytes et des monocytes, des porcelets issues des truies du lot C, dont l'alimentation a été complémentée selon l'invention, par rapport aux porcelets issus des truies du lot T.

La complémentation alimentaire selon la présente invention a également eu une influence sur la quantité d'anticorps dans le sang des porcelets comme l'indique la Figure 6. La concentration en IgG a été significativement accrue, et particulièrement suite à l'absorption du colostrum (Panneau A).

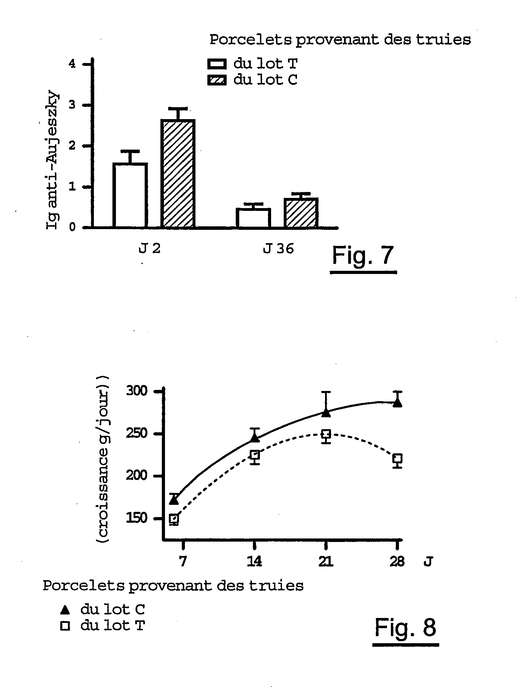

L'utilisation du complément alimentaire selon la présente invention s'est également traduit par une augmentation de la concentration en anticorps anti-Aujeszky dans le sang des porcelets issues des truies du lot C par rapport à ceux issus des truies du lot T, comme l'indique la figure 7.

On notera qu'une modification de la vitesse de croissance des porcelets a également été observée. En effet, les porcelets des truies du Lot C complémenté en huile de foie de requin ont eu une croissance moyenne plus importante que ceux provenant du lot T comme indiqué à la figure 8.

Ces résultats démontrent l'intérêt, dans le cadre de la production animale, de la complémentation alimentaire selon l'invention avec de l'huile de foie de poisson riche en *sn*-1-O-alkylglycérols. Ce régime particulier administré à des femelles en période de gestation puis de lactation a permis de modifier la composition du colostrum et d'influencer positivement l'immunité active (globules blancs) et passive (anticorps) des porcelets.

## Revendications

1. Complément alimentaire pour mammifères d'élevage en période de gestation puis, le cas échéant, en période de lactation destiné à modifier la composition lipidique et immunologique de leur colostrum puis, le cas échéant de leur lait **caractérisé en ce qu'**il comprend un extrait assimilable d'huile de foie de poisson(s) contenant au moins un composé *sn*-1-O-alkylglycérol.

2. Complément alimentaire selon la revendication 1 **caractérisé en ce** ledit composé sn-1-O-alkylglycérol est le 16: 1n-7 *sn*-1-O-alkylglycérol.

3. Complément alimentaire selon la revendication 2 **caractérisé en ce qu'**il comprend un mélange de 16:0 *sn*-1-O-alkylglycérol, 16:1n-7 *sn*-1-O-alkylglycérol, 18:1n-9*sn*-1-O-alkylglycérol et 18:1n-7 *sn*-1-O-alkylglycérol,

4. Complément alimentaire selon l'une quelconque des revendications 1 à 3 **caractérisé en ce que** ladite huile de foie de poisson est une huile de foie de requin(s).

5. Complément alimentaire selon la revendication 4 **caractérisé en ce que** ledit requin est *Centrophorus squamosus*.

6. Complément alimentaire selon la revendication 5 **caractérisé en ce que** ladite huile de foie de poisson(s) titre à au moins 20 % en poids en composé(s) *sn-*1-O-alkylglycérol(s).

7. Complément alimentaire selon l'une quelconque des revendications 1 à 6 **caractérisé en ce qu'**il est destiné aux truies.

## Claims

1. Food supplement for livestock during the gestation period then, where applicable, during the lactation period intended to modify the lipidic and immunological composition of their colostrum then, where applicable, their milk, **characterised in that** it comprises an easily absorbed extract of fish liver oil containing at least one compound *sn*-1-O-alkylglycerol.

2. Food supplement according to Claim 1, **characterised in that** said compound *sn-*1-O-alkylglycerol is 16:1n-7 *sn*-1-O-alkylglycerol.

3. Food supplement according to Claim 2, **characterised in that** it comprises a mixture of 16:0 *sn*-1-O-alkylglycerol, 16:1n-7 *sn*-1-O-alkylglycerol, 18:1n-9 *sn*-1-O-alkylglycerol and 18:1n-7 *sn*-1-O-alkylglycerol.

4. Food supplement according to any one of Claims 1 to 3, **characterised in that** said fish liver oil is a shark liver oil.

5. Food supplement according to Claim 4, **characterised in that** said shark is *Centrophorus squamosus*.

6. Food supplement according to Claim 5, **characterised in that** said fish liver oil titrates to at least 20 % by weight of compound(s) *sn*-1-O-alkylglycerol.

7. Food supplement according to any one of Claims 1 to 6, **characterised in that** it is intended for sows.

## Patentansprüche

1. Futtermittelergänzung für Zuchtsäugetiere in der Trächtigkeitsperiode und gegebenenfalls in der Säugeperiode, vorgesehen, die Lipidzusammensetzung und immunologische Zusammensetzung von deren Kolostrum und gegebenenfalls von deren Milch zu modifizieren, **dadurch gekennzeichnet, daß** es einen assimilierbaren Fischleberölextrakt umfaßt, der wenigstens eine *sn*-1-O-Alkylglyzerinverbindung enthält.

2. Futtermittelergänzung nach Anspruch 1, **dadurch gekennzeichnet, daß** die sn-1-O-Alkylglyzerinverbindung 16:ln-7 *sn*-1-O-Alkylglyzerin ist.

3. Futtermittelergänzung nach Anspruch 2, **dadurch gekennzeichnet, daß** sie ein Gemisch von 16:0 *sn*-1-O-Alkylglyzerin, 16:ln-7 *sn*-1-O-Alkylglyzerin, 18:ln-9 *sn*-1-O-Alkylglyzerin und 18:1n-7 sn1-O-Alkylglyzerin umfaßt.

4. Futtermittelergänzung nach einem der Ansprüche 1 - 3, **dadurch gekennzeichnet, daß** das Fischleberöl Haifischleberöl ist.

5. Futtermittelergänzung nach Anspruch 4, **dadurch gekennzeichnet, daß** der Hai ein *Centrophorus squamosus* ist.

6. Futtermittelergänzung nach Anspruch 5, **dadurch gekennzeichnet, daß** dieses Fischleberöl wenigstens 20 Gewichtsprozent an *sn*-1-O-Alkylglyzerinverbindung(en) hat.

7. Futtermittelergänzung nach einem der Ansprüche 1 - 6, **dadurch gekennzeichnet, daß** es für Schweine vorgesehen ist.
